# EUROPÄISCHE PATENTANMELDUNG

(11) **EP 1 557 162 A1**
(43) Veröffentlichungstag der Anmeldung: **27.07.2005**
(21) Anmeldenummer: 04026134.9
(22) Anmeldetag: 04.11.2004
(51) Int. Cl.: A61K 9/70, A61K 31/4545, A61K 31/495, A61P 37/08, A61L 24/00, A61L 24/06

(54) **Pflastersystem zur Verabreichung von Antihistaminika**

(30) Priorität: 19.12.2003 DE 10360592
(71) Anmelder: Beiersdorf AG, 20253 Hamburg (DE)
(72) Erfinder: Nierle, Jens, Dr., 21147 Hamburg (DE); Zwiener, Thorben, 20251 Hamburg (DE)

(57) **Zusammenfassung**

Gegenstand der Erfindung ist eine lösemittelfreie Polymermatrix als Basis für ein transdermales therapeutisches System (TTS), mit dem es möglich ist, sowohl Loratadin als auch Ceterizin als pharmazeutische Wirkstoffe transdermal zu applizieren, um somit die Symptome einer allergischen Erkrankung zu beseitigen.

## Beschreibung

Gegenstand der Erfindung ist eine lösemittelfreie Polymermatrix als Basis für ein transdermales therapeutisches System (TTS), mit dem es möglich ist, sowohl Loratadin als auch Ceterizin als pharmazeutische Wirkstoffe transdermal zu applizieren, um somit die Symptome einer allergischen Erkrankung zu beseitigen.

Bei der Formulierung von Arzneimitteln zur topischen Applikation von Wirkstoffen kommt der Wahl der Hilfsstoffe entscheidende Bedeutung zu. So ist bekannt, dass ein Zusammenhang zwischen Löslichkeit eines Wirkstoffs in einem Vehikel und seiner Freisetzung aus diesem Vehikel besteht. Wird ein wirkstoffhaltiges Vehikel auf die Haut appliziert, dann stellt sich ein Verteilungsgleichgewicht des Wirkstoffs zwischen Vehikel und Haut ein. Die Lage dieses Gleichgewichts wird von der Löslichkeit in beiden Phasen bestimmt.

Transdermal Therapeutische Systeme (TTS) zur Abgabe von Wirkstoffen durch die Haut sind seit langer Zeit bekannt. Die topische Applikation von Arzneimitteln über wirkstoffhaltige Pflastersysteme bietet zwei Hauptvorteile: Erstens wird durch diese Darreichungsform eine Freisetzungskinetik des Wirkstoffes erster Ordnung realisiert, wodurch über einen sehr langen Zeitraum ein konstanter Wirkstoffspiegel im Organismus aufrechterhalten werden kann. Zweitens werden über den Aufnahmeweg durch die Haut der Magen-Darm-Trakt sowie die erste Leberpassage vermieden. Dadurch können ausgewählte Arzneistoffe in einer geringen Dosierung wirkungsvoll verabreicht werden. Dies ist insbesondere dann von Vorteil, wenn eine lokale Wirkung des Arzneistoffes unter Umgehung einer systemischen Wirkung erwünscht ist. Dies ist zum Beispiel bei der Behandlung rheumatischer Gelenkbeschwerden oder Muskelentzündungen der Fall.

Eine in der Fachliteratur gut beschriebene Ausführungsform solcher transdermalen Systeme stellen Matrixsysteme oder monolitische Systeme dar, in denen der Arzneistoff direkt in den druckempfindlichen Haftklebstoff eingearbeitet wird. Eine solche haftklebrige, wirkstoffhaltige Matrix ist im anwendungsfertigen Produkt auf der einen Seite mit einem für den Wirkstoff undurchlässigen Träger ausgestattet, auf der gegenüberliegenden Seite befindet sich eine mit einer Trennschicht ausgestattete Trägerfolie, die vor der Applikation auf die Haut entfernt wird (kleben&dichten, Nr.42, 1998, S. 26 bis 30). So wird vornehmlich die Verwendung von Polyacrylaten und/oder Polyurethanen als Basis der haftklebrigen Polymermatrix erwähnt (Lamba, Woodhouse, Cooper, "Polyurethanes in Biomedical Applications", CRC Press, 1998, S. 240) und WO 01/68060.

Ein Problem bei der Herstellung transdermaler therapeutischer Systeme ist das Einbringen polarer Wirkstoffe in die zumeist unpolaren Polymermatrizes. Dadurch können bevorzugte Wirkstoffe mitunter nur schlecht oder nur in begrenzter Konzentration in die Polymermatrix eingearbeitet werden. Des weiteren besteht die Gefahr, dass aufgrund der unterschiedlichen Polarität und Unlöslichkeit der Wirkstoffe in der Polymermatrix die Wirkstoffe mit der Zeit aus dem Polymersystem auskristallisieren. Eine Langzeitstabilität ist damit nicht gewährleistet.
Einen Vorschlag zur Lösung dieser Probleme wird durch den Zusatz von Lösemittel gegeben. Die Einarbeitung von Lösemittel in eine polymere Matrix hat aber verschiedene Nachteile. Zu erwähnen sind hier die Schädlichkeit der meisten organischen Lösungsmittel, hohe technische Aufwände für Absaugung und Wiedergewinnung, hohe Kosten für erforderliche hochreine Lösungsmittel und besonders ein sehr hoher Aufwand zur Entfernung von Lösungsmittelresten aus der Matrix.

Diese Lösemittel werden ebenfalls durch die Haut resorbiert, wodurch sich der Lösemittelgehalt in der TTS-Matrix verringert. Weiterhin wurde gefunden, dass von der Haut abgegebenes Wasser sich im Lösungsmittel anreichert, da es über die hydrophobe Polymermatrix schlecht gebunden werden kann. Beide Mechanismen führen dazu, dass die Wirkstofffreisetzung ungünstig beeinflusst wird.

WO 01/68060 A2 beschreibt ein transdermales therapeutisches System vom Matrix-Typ, wobei den hydrophoben Basispolymeren der wirkstoffhaltigen Polymermatrix Polyacrylatpolymere beigemischt werden. Die hydrophoben Basispolymere können ausgewählt werden aus der Gruppe der Polysiloxane, Polyisobutylen, Polyisopren, Styrol-Dien-Styrol-Blockcopolymer oder Mischungen daraus. Die Polyacrylatpolymere sollen den Nachteil der Übersättigung des Wirkstoffs im TTS durch die Lösungsmittelzugabe als auch eine Rekristallisierung vor der Aufnahme durch die Haut vermeiden.

EP 794770 beschreibt acrylatbasierende transdermale Systeme in denen Loratidin als Wirkstoff enthalten ist. Auch hier erfolgt die Herstellung über Lösemittel und es liegen damit in der Polymermatrix Restlösemittel vor, die zu einer Belastung des Anwenders führen können.

Aufgabe der vorliegenden Erfindung ist es ein lösemittelfreies Matrixsystem bereit zu stellen, dass Antihistaminika enthält und als TTS angewendet werden kann.

An wirkstoffhaltige Pflastersysteme werden neben einer kontrollierten Wirkstofffreisetzung aber auch bestimmte Anforderungen an die Klebmatrix, wie beispielsweise Hautfreundlichkeit, gutes Haftvermögen über eine lange Anwendungsdauer und eine schmerzfreie Entfernbarkeit gestellt. Eine häufig beobachtete Nebenwirkung insbesondere bei Polyacrylatklebmatrizes ist das Auftreten von Hautirritationen, die besonders bei längerer oder wiederholter Applikation eines wirkstoffhaltigen Pflasters an einer gleichbleibenden Körperregion auftreten. Sie werden hauptsächlich durch die Inhaltsstoffe der haftklebrigen Matrix verursacht. Auch ein schmerzhaftes Wiederablösen des wirkstoffhaltigen Pflasters nach längerer Tragezeit wird häufig beobachtet. Es sollte daher auf den Zusatz weiterer Klebstoffe, die unter Umständen Nebenwirkungen wie Mazeration, Allergien u.a. hervorrufen, verzichtet werden.
Wirkstoffhaltige Pflaster werden in der Regel auf gesunder, intakter Haut appliziert. Gerade hier ist es besonders wichtig, die intakte Haut nicht durch ein Arzneimittel zu reizen oder gar zu schädigen. Eine genügende Kohäsivität ist zusätzlich notwendig, um das wirkstoffhaltige Pflaster nach beendeter Tragedauer rückstandsfrei und insbesondere auch schmerzfrei entfernen zu können.

Aufgabe der vorliegenden Erfindung ist es daher auch ein transdermales therapeutisches System bereit zu stellen, das dem Anwender den bestmöglichen Komfort, d.h. lange Tragedauer, hautschonende Applikation und schmerzfreie Wiederablösung, ermöglicht.

Gelöst werden die angeführten Aufgaben durch ein Matrixsystem entsprechend dem Hauptanspruch. Gegenstand der Unteransprüche sind vorteilhafte Ausführungsformen des erfindungsgemäßen Systems. Des weiteren umfasst die Erfindung die Verwendung derartiger Systeme, insbesondere als TTS.

Es war überraschend und für den Fachmann nicht vorauszusehen und darin liegt die Lösung dieser Aufgaben, dass ein wirkstoffhaltiges, lösemittelfreies Matrixsystem auf einer PIB- und/oder SIS- Kautschuk basierenden Haftklebemasse enthaltend Loratadin und/oder Ceterizin, die Aufgaben löst.

Als Polymermatrizes werden solche Massen bevorzugt, die gänzlich ohne Lösungsmittel auskommen. Besonders bevorzugt sind sog. hot-melt Massen. Heißschmelzklebemassen erlauben die Vermeidung von Nachteilen, die mit dem bei herkömmlichen wirkstoffhaltigen Klebemassen notwendigen Einsatz von Lösemitteln verbunden sind. Als Heißschmelzklebemassen lassen sich vorteilhafterweise thermoplastische Heißschmelzselbstklebemassen auf Basis natürlicher und synthetischer Kautschuke und anderer synthetischer Polymere wie Polyisobutylene oder Styrol/Isopren/Styrol-Triblockcopolymere einsetzen. Zusatzstoffe wie Klebharze, Weichmacher, Stabilisatoren und andere Hilfsstoffe können soweit erforderlich zugesetzt werden.
Die Vorteile der Heißschmelzklebemassen bei der Herstellung von wirkstoffhaltigen Klebemassen werden schon seit einigen Jahren in der Patentliteratur beschrieben (siehe zum Beispiel EP 0 305 757 A1).
Bevorzugt ist die Verwendung von Polyisobutylen (PIB) oder Styrol/Isopren/Styrol-Triblockcopolymeren (SIS).

Um die funktionsgerechte Verwendung sicherzustellen, können die Heißschmelzklebemassen geschäumt sein, und zwar vorzugsweise geschäumt mit inerten Gasen wie Stickstoff, Kohlendioxid, Edelgasen, Kohlenwasserstoffen oder Luft oder deren Gemischen. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.% betragen und kann bis zu etwa 50 Vol.% reichen. In der Praxis haben sich Werte von 10 Vol.% bis 35 Vol.%, bevorzugt 15 Vol.%, gut bewährt. Wird bei relativ hohen Temperaturen von ungefähr 100 °C und vergleichsweise hohem Innendruck gearbeitet, entstehen sehr offenporige Klebstoffschaumschichten, die besonders gut luft- und wasserdampfdurchlässig sind. Ein Schäumungsgrad von über 50 Vol.% wird dabei als technisch unpraktikabel angesehen, da die Kohäsion der Masse sich dadurch verringert.

Aus der Vielzahl bekannter Polymermatrizes sind Polyisobutylene besonders bevorzugt. Polyisobutylene erfüllen als Matrixgrundlage die Anforderungen einer selbstklebenden, hautschonenden und schmerzfrei ablösbaren Polymermatrix besonders gut, so dass es folgerichtig ist, die Polyisobutylene bevorzugt als Matrixgrundlage auszuwählen.

Es bestand das Bedürfnis den antihistaminischen Effekt wirkstoffhaltiger TTS zu verbessern und eine systematische Applikation zu erreichen. Probleme bestanden jedoch in der schonenden Einarbeitung von Wirkstoffen in die Polymermatrix, insbesondere wenn es sich um lösemittelbasierende Massen handelte. Da dort durch die abschließenden Trocknungsprozesse der Wirkstoff hohen thermischen Belastungen ausgesetzt wird. Als Antihistaminika kommen erfindungsgemäß Loratadin und Ceterizin in Frage.

Loratadin, internationale Freiname für das Antihistaminikum Ethyl-4-(8-chlor-5,6-dihydro-11*H*-benzo[5,6]cyclohepta[1,2-b]pyridin-11-yliden)-1-piperidincarboxylat, C₂₂H₂₃ClN₂O₂, ist mit der Formel 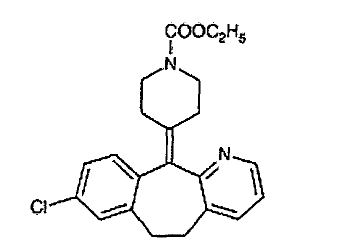 bekannt. Es wurde 1981 von Schering patentiert und ist beispielsweise von Essex Pharma (Lisino®) im Handel erhältlich.

Ceterizin ist ein weiteres Antihistaminikum mit der Formel

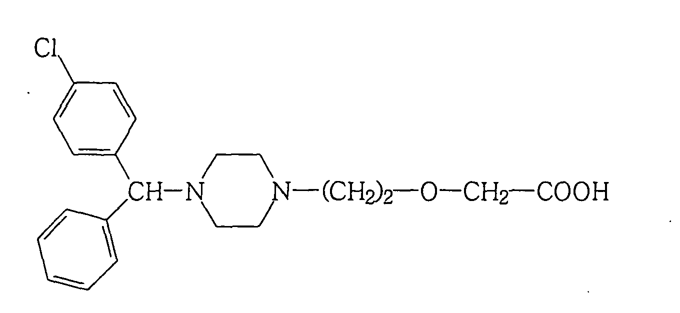

Die Wirkstoffe sind vorteilhaft zu einem Anteil von 0,1 bis 10 Gew.%, bevorzugt 1 bis 4 Gew.%, bezogen auf die Gesamtmasse der Matrix, enthalten. Als besonders bevorzugt zeigt sich, wenn der Wirkstoff in Diethylenglykol gelöst vorliegt und so in die Polymermatrix eingearbeitet wird.

Ein transdermales therapeutisches System (TTS), zumeist in Form eines Pflasters, umfasst eine erfindungsgemäße selbstklebende wirkstoffhaltige Polymermatrix, eine wirkstoffundurchlässige Rückschicht und eine ablösbare Schutzschicht, die vor dem Applizieren auf der Haut entfernt wird. Weitere Ingredienzien, wie Füllstoffe, Stabilisatoren, Enhancer und/oder kosmetische Zusätze können in der Polymermatrix eingebaut werden, um das TTS an die unterschiedlichen Anwendungsgebiete anzupassen und um ein anwendungsfreundliches TTS bereit zu stellen. Die erfindungsgemäße Polymermatrix kann selbstklebend oder nicht klebend ausgeführt sein. In letzterem Fall, beispielsweise einem sog. Island Dressing, sollten jedoch Hilfsmittel für die Befestigung auf der Haut gegeben sein.
Besonders vorteilhaft ist die erfindungsgemäße Polymermatrix auf einer flexiblen Deckschicht aufgebracht, insbesondere bei der Verwendung als selbstklebendes Pflaster.

Aufgebaut ist ein entsprechendes Pflaster aus einem Träger wie Folien, Vliese, Gewebe, Schäume etc., der Klebmatrix und Abdeckfolie, Abdeckpapier oder Trennpapier zum Schutz der klebenden Matrix vor dem Gebrauch des Pflasters.
In einer weiteren bevorzugten Ausführungsform der Erfindung werden als Träger Polymerfolien, Vliese, Gewebe sowie deren Kombinationen eingesetzt. Als Trägermaterialien stehen u.a. Polymere wie Polyethylen, Polypropylen und Polyurethan oder auch Naturfasern zur Auswahl.

Zusammenfassend kann festgehalten werden, dass als Trägermaterialien sich alle starren und elastischen Flächengebilde aus synthetischen und natürlichen Rohstoffen eignen. Bevorzugt sind Trägermaterialien, die so eingesetzt werden können, dass sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt. Weiter können diese Materialien vor- beziehungsweise nachbehandelt werden. Gängige Vorbehandlungen sind Corona und Hydrophobieren; geläufige Nachbehandlungen sind Kalandern, Tempern, Kaschieren, Stanzen und Eindecken.
Besonders vorteilhaft ist, wenn das Trägermaterial sterilisierbar, bevorzugt γ-(gamma) sterilisierbar, ist.
Die genannten Eigenschaften der Klebmatrix legen insbesondere die Verwendung für medizinische Produkte, insbesondere Pflaster, medizinische Fixierungen, Wundabdeckungen, orthopädische oder phlebologische Bandagen und Binden nahe.

Schließlich kann die Polymermatrix mit einem klebstoffabweisenden Trägermaterial, wie silikonisiertem Papier, eingedeckt oder mit einer Wundauflage oder einer Polsterung versehen werden. Auf seiner selbstklebend ausgerüsteten, später der Haut zugewandten Seite ist das erfindungsgemäße Pflaster über seine ganze Breite bis zum Gebrauch üblicherweise mit einem klebstoffabweisenden Trägermaterial abgedeckt. Dieses schützt die Selbstklebeschicht aus der gut hautverträglichen Klebemasse der Matrix, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.
Weitere Ausführungsformen können dergestalt sein, dass das TTS einschichtig oder mehrschichtig selbstklebend aufgebaut ist. Weiterhin kann zwischen der Rückseite der Matrix und dem Abdeckträger sich eine zweite erfindungsgemäße Polymermatrix mit höherer Wirkstofflöslichkeit als Reservoir befinden.
Auf der Klebseite der Matrix befindet sich teilweise (z.B. am Rand) eine zweite Matrix mit hoher Klebkraft zur zusätzlichen Fixierung, aber ungenügender bzw. unnötiger Wirkstofflöslichkeit.
Die wirkstofffreie Matrix befindet sich zwischen zwei nicht verankernden Folien und wird zur Fixierung von Elektroden etc., oder wegen der Wasseraufnahmefähigkeit bei entsprechender Geometrie von Colostomie-/Ileostomiebeuteln genutzt.

Die Herstellung der mit Wirkstoff dotierten Haftklebemasse erfolgt im so genannten 'Hotmelt-Verfahren', bei dem die einzelnen Zusatzstoffe nacheinander einem Kneter oder Extruder zugegeben werden und in diesem homogen durchmischt werden. Ein Vorteil dieser Methode besteht darin, dass keine Restlösemittel in der Haftklebemasse verbleiben, welche ein erhöhtes Gesundheitsrisiko für den Anwender darstellen. Einen weiteren Vorteil stellt die extrem kurze thermische Belastung des Wirkstoffes im Extrusionsprozess dar, da die vergleichsweise lange Trockenzeit der auf Lösemittel basierenden Herstellung entfällt.

Die Herstellung erfolgt lösungsmittelfrei. Sowohl in einem diskontinuierlichen Prozess in einem Kneter als auch kontinuierlich in einem Extruder lassen sich die Polymermassen herstellen.
Bei der Herstellung im Kneter wird das hochpolymere Material vorgelegt. Das Material wird beispielsweise bei 20 U/min bei 50,0°C vorgeknetet. Anschließend werden die übrigen Materialien nacheinander zudosiert. Nach dem Ende des Knetvorgangs wird die Masse aus dem Kneter ausgetragen und kann beschichtet werden.
Bei der Extrusion der Masse wird zunächst das hochpolymere Material über die Einzugszone zudosiert und aufgeschlossen. Anschließend werden die übrigen Komponenten der Reihe nach zudosiert, wobei auch hier zunächst die festen und anschließend die flüssigen Komponenten zugegeben werden. Die Zudosierung des Wirkstoffs erfolgt wieder als letzte Komponente. Im Gegensatz zum Knetprozess wird bei der Extrusion Inline beschichtet.

Die nachfolgenden Beispiele veranschaulichen die Erfindung ohne sie einzuschränken und führen die erfindungsgemäßen Polymermatrizes, sowie deren Herstellung auf:

### Beispiel 1

Vorangehend werden 3,80g (2% w/w) Loratadin in 5,70g (3,0% w/w) Diethylenglykol gelöst.
In einem IKA Messkneter werden 51,30g (27,00% w/w) Vistanex LM MH, 25,65g (13,50% w/w) Vistanex MM L80 und 21,85g (11,50% w/w) Eastoflex PLS E1003D bei 70 °C 15 min geknetet. Es werden 64,60g (34% w/w) Elcema P020 (pulvrige Cellulose) hinzugefügt und 60 min geknetet. Anschließend fügt man 11,40g (6,0% w/w) Cetiol V, 5,70g (3,00% w/w) Kollidon 30 und dann das in Diethylenglykol gelöste Loratadin der Mischung zu und homogenisiert weitere 60 min.
Die fertige Masse wird dann zwischen Trennpapier mit einer Hydraulikpresse ausgepresst und anschließend auf Träger und Trennfolie umkaschiert.

### Beispiel 2

In einem Becherglas werden 20,0g (50% w/w) Dimethylsulfoxid (DMSO) vorgelegt und auf 70°C erwärmt. Unter Rühren werden 20,00g (50% w/w) Kollidon 30 hinzugefügt. Die Mischung wird 60 min bei 70°C gerührt. Die fertige Mischung liegt als Gel vor, wird abgekühlt und in ein Vorratsgefäß gefüllt.

### Beispiel 3

In einem IKA Messkneter werden 66,50g (35% w/w) Vector 4113, 30,40g (16,00% w/w) Harz SE10 und 9,50g (5% w/w) Harz 95 bei 100°C zwei Stunden geknetet. Es werden 57,00g (30% w/w) Elcema P020 (pulvrige Cellulose) hinzugefügt und 60 min geknetet. Anschließend fügt man 19,00g (10% w/w) Eutanol G und 3,80g (2,0% w/w) des DMSO/Kollidon 30 Gels (Beispiel 2) der Mischung zu und knetet weitere 5 min. Danach erfolgt die Zugabe von 3,80g (2,0% w/w) Loratadin. Die resultierende Mischung wird weitere 60 min im Kneter homogenisiert.
Die fertige Masse wird dann zwischen Trennpapier mit einer Hydraulikpresse ausgepresst und anschließend auf Träger und Trennfolie umkaschiert.

### Beispiel 4

Entsprechend Beispiel 3, nur ist Loratadin durch Ceterizin ersetzt worden.

### Beispiel 5 (Extrusion)

Die Herstellung der Masse erfolgt in einem 50mm Leistritz Doppelschneckenextruder mit einem Masseausstoss von 50kg/h. Das Temperaturprofil für den Prozess beträgt 130-100°C von der Aufgabezone bis zur Austragszone. Die Inhaltsstoffe der Klebmatrix werden nacheinander entlang der Gesamtlänge des Verfahrensteils in folgender Reihenfolge zudosiert:

| | | | |
|---|---|---|---|
| 1. | Vistanex MM L80: | 5,5 kg/h | granuliert |
| 2. | Vistanex LM MH: | 13,0 kg/h | Schmelze |
| 3. | Eastoflex PLS E1003D: | 7,0 kg/h | Schmelze |
| 4. | pulvrige Cellulose: | 16,0 kg/h | Feststoff |
| 5. | Kollidon 30/ DMSO: | 1,0 kg/h | flüssig |
| 5. | Cetiol V: | 5,0 kg/h | flüssig |
| 6. | Wirkstoff: | 2,5 kg/h | Feststoff |

Die Masse wird kontinuierlich hergestellt und aus einer Breitschlitzdüse ausgetragen. Zwischen Austragszone und Breitschlitzdüse ist eine Zahnradpumpe installiert, die für einen gleichmäßigen Masseaustrag sorgt.
Die Klebmasse wird anschließend über einen Kalander zwischen Trägermaterial und einer Trennfolie beschichtet und zur weiteren Verarbeitung auf Rollen aufgewickelt.

Bei den zitierten Stoffen handelt es sich:

| | |
|---|---|
| Kollidon 30 | Polyvinylpyrrolidon MW: 44.000 bis 54.000 |
| Vistanex LM MH | niedermolekulares Polyisobutylen MW: 12.000- 53.000 |
| Vistanex MM L80 | hochmolekulares Polyisobutylen MW: 750.000- 1.050.000 |
| Eastoflex PLS E1003D | amorphes α-Polyolefin |
| Cetiol V | Decyloleat |
| Eutanol G | Oktyldodecanol |

Aufgebaut ist ein entsprechendes Pflaster aus einem Träger wie Folien, Vliesse, Gewebe, Schäume etc., der Klebmatrix und Abdeckfolie, Abdeckpapier oder Trennpapier zum Schutz der klebenden Matrix vor dem Gebrauch des Pflasters.

## Patentansprüche

1. Wirkstoffhaltiges, lösemittelfreies Matrixsystem aus einer Polyisobutylen (PIB) oder Styrol/Isopren/Styrol-Triblockcopolymeren (SIS) basierenden Haftklebemasse enthaltend Loratadin und/oder Ceterizin.

2. Polymermatrix nach Anspruch 1, **dadurch gekennzeichnet, dass** die Matrix als hydrophobe Basispolymere Polyisobutylen enthält.

3. Polymermatrix nach Anspruch 1 oder 2 enthaltend den Wirkstoff zu einem Anteil von 0,1 bis 10 Gew.%, bevorzugt 1 bis 4 Gew.%, bezogen auf die Gesamtmasse der Matrix.

4. Polymermatrix nach einem der vorstehenden Ansprüche, **dadurch gekennzeichnet, dass** der Wirkstoff in Diethylenglykol gelöst vorliegt.

5. Verfahren zur Herstellung einer lösemittelfreien Polymermatrix nach einem der Ansprüche 1 bis 4 in einem diskontinuierlichen Knetprozess.

6. Verfahren zur Herstellung einer lösemittelfreien Polymermatrix nach einem der Ansprüche 1 bis 4 in einem kontinuierlichen Extrusionsprozess.

7. Verwendung der Polymermatrix nach einem der Ansprüche 1 bis 4 zur Herstellung eines transdermalen therapeutischen Systems (TTS).

8. Verwendung der Polymermatrix nach einem der Ansprüche 1 bis 4 zur transdermalen Applikation von Loratadin und/oder Ceterizin.
